# EUROPÄISCHE PATENTANMELDUNG

(11) **EP 4 293 457 A1**
(43) Veröffentlichungstag der Anmeldung: **20.12.2023**
(21) Anmeldenummer: 22179239.3
(22) Anmeldetag: 15.06.2022
(51) Int. Cl.: G05B 23/02, G06N 3/04

(54) **ÜBERWACHUNGSVERFAHREN, COMPUTERPROGRAMMPRODUKT, ÜBERWACHUNGSSEINHEIT, GASANALYSEVORRICHTUNG UND VERWENDUNG EINER KÜNSTLICHEN INTELLIGENZ**

(71) Anmelder: Siemens Aktiengesellschaft, 80333 München (DE)
(72) Erfinder: Dr. EDER, Jana, 1180 WIEN (AT); WEILANDT, Simon, 6228 Karlsruhe (DE); BITTER, Ralf, 76356 Weingarten (DE); HOLLY, Stephanie, 2000 Stockerau (AT); OFFERMANN, Tim, 76764 Rheinzabern (AT); Dr. SCHALL, Daniel, 2020 Hollabrunn (AT); STRAUCH, Piotr, 76761 Rülzheim (DE)
(74) Vertreter: Siemens Patent Attorneys

(57) **Zusammenfassung**

Die Erfindung betrifft Überwachungsverfahren (100, 200) für eine Anlage (10) mit einer Mehrzahl an Geräten (12), die dazu ausgebildet sind, jeweils einen zugehörigen Messwert (15) und/oder einen Steuerbefehl (17) bereitzustellen. Die Überwachungsverfahren (100, 200) basieren auf der Verwendung einer Weiterverarbeitungsvorrichtung (40) bzw. eines Neuronalen Netzes (80), mit denen Messwerte (15) bzw. Steuerbefehle (17) ausgewertet werden. Die Erfindung betrifft auch ein Computerprogrammprodukt (70), mit denen die Überwachungsverfahren (100, 200) durchführbar sind und eine entsprechend ausgestattete Überwachungseinheit (75). Weiter betrifft die Erfindung eine Gasanalysevorrichtung (11), mit einer derartigen Überwachungseinheit (75) ausgestattet ist. Ferner betrifft die Erfindung eine Verwendung einer Künstlichen Intelligenz (99) zum Überwachen einer Gasanalysevorrichtung (11).

## Beschreibung

Die Erfindung betrifft ein Überwachungsverfahren zum Überwachen einer Anlage und ein dazu ausgebildetes Computerprogrammprodukt. Weiter betrifft die Erfindung eine Überwachungseinheit, die über ein entsprechendes Computerprogrammprodukt verfügt, und eine Gasanalysevorrichtung mit einer derartigen Überwachungseinheit. Ferner betrifft die Erfindung die Verwendung einer Künstlichen Intelligenz zum Überwachen einer Gasanalysevorrichtung.

Die Druckschrift KR 2021147318 A offenbart eine multisensorbasierte Künstliche Intelligenz, die zu einer Fehlerdiagnose in einem mechanischen Gerät ausgebildet ist. Darin werden Sensordaten gesammelt und mittels eines Autoencoders verarbeitet.

Aus der Anmeldung US 2020/0310370 A1 ist ein Steuerungssystem mit einem Autoencoder bekannt, in dem Sensordaten zu abgeleiteten Sensordaten verarbeitet werden. Der Autoencoder empfängt die abgeleiteten Sensordaten und hieraus eine Prognose ermittelt. Basierend auf der Prognose wird eine Steuerungseingriff ausgelöst.

In unterschiedlichen Anwendungen der Automatisierungstechnik werden zunehmend komplexe Anlagen mit einer steigenden Anzahl an einzelnen Geräten eingesetzt. Ebenso wird ein flexibler Betrieb zur Verarbeitung unterschiedlicher Eingangsstoffe angestrebt. Aufgrund der steigenden Betriebs- und Anlagenkomplexität wird eine Überwachung solcher Anlagen anspruchsvoller. Es besteht damit Bedarf an einer Möglichkeit zur Überwachung komplexer Anlagen, die eine zuverlässige selbsttätige Fehlererkennung bietet und schnell auf wechselnde Betriebsanforderungen anpassbar ist. Dies gilt insbesondere für Gasanalysevorrichtungen, die aufgrund steigender Anforderungen an die Messgenauigkeit zunehmend komplexer und empfindlicher werden.

Die Aufgabenstellung wird durch ein erfindungsgemäßes Überwachungsverfahren gelöst, das dazu ausgebildet ist, einen Betrieb einer Anlage zu überwachen. Die Anlage umfasst eine Mehrzahl an Geräten, die dazu ausgebildet sind, jeweils einen Messwert und/oder einen Steuerbefehl bereitzustellen. Die Geräte können beispielsweise Sensoren, Steuereinheiten, Regelungseinheiten oder Kombinationen hieraus sein. Die Messwerte können erfasste physikalische Größen sein oder Zustandsangaben über das jeweilige Gerät sein. Das Überwachungsverfahren umfasst einen ersten Schritt, in dem ein Eingangssignal-Array bereitgestellt wird, das eine Mehrzahl an Zellen umfasst. Eine Zelle des Eingangssignal-Arrays ist dazu geeignet, einen vorgegebenen Messwert bzw. Steuerbefehl zu empfangen und einer Weiterverarbeitungseinheit zuzuführen. Das Eingangssignal-Array kann als ein- zwei- oder höherdimensionales Array, also Datenfeld, ausgebildet sein. Weiter wird im ersten Schritt ein Überwachungsdaten-Array bereitgestellt, das in puncto Dimensionalität und Größe, also Anzahl an Zellen, dem Eingangssignal-Array entspricht. Jeweils eine Zelle des Eingangssignal-Arrays korrespondiert mit einer Zelle des Überwachungsdaten-Arrays.

Ebenso weist das Überwachungsverfahren einen zweiten Schritt auf, in dem die Anlage in einem aktiven Betriebszustand bereitgestellt wird. Im aktiven Betriebszustand werden im Wesentlichen durchgängig Messwerte bzw. Steuerbefehle erzeugt und für das Überwachungsverfahren zur Weiterverarbeitung bereitgestellt. Die erfassten Messwerte und/oder Steuerbefehle werden an das Eingangssignal-Array übergeben und in die korrespondierenden zugehörigen Zellen als Inhalte eingespeist. Über die Weiterverarbeitungseinheit werden diese zu Inhalten von korrespondierenden Zellen im Überwachungsdaten-Array verarbeitet.

In einem dritten Schritt wird basierend auf den im zweiten Schritt in das Eingangssignal-Array ein System-Abweichungsparameter ermittelt. Der System-Abweichungsparameter ist durch eine Mehrzahl an Zellen des Überwachungsdaten-Arrays gebildet. Der System-Abweichungsparameter kann beispielsweise als eine Summe von Inhalten von Zellen des Überwachungsdaten-Arrays oder als eine Summe von quadrierten Inhalten der Zellen des Überwachungsdaten-Arrays ausgebildet sein. Die Inhalte der Zellen des Überwachungsdaten-Arrays können jeweils einzeln oder mehrere zusammen auf einen Referenzwert normiert sein. Die Inhalte sind typenkompatibel zu Inhalten der korrespondierenden Zellen des Eingangssignal-Arrays ausgebildet. Ein Gewichten einzelner Zellen des Überwachungsdaten-Arrays ist ebenso möglich. Das Gewichten kann durch einen Algorithmus vor oder während des Ablaufs des Überwachungsverfahrens durchgeführt werden. Wenn der System-Abweichungsparameter im dritten Schritt einen einstellbaren System-Schwellenwert übersteigt, wird das Vorliegen eines bestimmungswidrigen Zustands erkannt.

Ferner weist das Überwachungsverfahren einen vierten Schritt auf, der durchführbar ist, wenn im dritten Schritt ein bestimmungswidriger Betriebszustand der Anlage erkannt wird. Darin werden die im System-Abweichungsparameter kombinierten Inhalte der Zellen des Überwachungsdaten-Arrays separat weiterverarbeitet. Anhand von Inhalten der Zellen des Überwachungsdaten-Arrays und Inhalten von korrespondierenden Zellen des Eingangssignal-Arrays werden paarweise Zellen-Abweichungsparameter ermittelt. Dies kann beispielsweise durch eine Differenzbildung erfolgen. Weiter wird im vierten Schritt eine Zelle des Eingangssignal-Arrays oder des Überwachungsdaten-Arrays als Defekt-Zelle erkannt, wenn ein dazugehöriger Zellen-Abweichungsparameter einen einstellbaren Zellen-Schwellenwert betragsmäßig übersteigt. Der Zellen-Schwellenwert kann durch einen Benutzer, eine Tabelle, einen Algorithmus, oder eine Künstliche Intelligenz vorgegeben sein. Die Defekt-Zelle kann dabei eine Zelle des Eingangssignal-Arrays oder eine Zelle des Überwachungsdaten-Arrays sein. Somit ist ein zugehöriges Gerät als defekt identifizierbar. Insbesondere kann auch eine Komponente eines Geräts als defekt identifiziert werden. Ferner wird im erfindungsgemäßen Überwachungsverfahren eine Warnung ausgegeben, wenn eine Defekt-Zelle erkannt wird. Die Warnung kann als visuelle oder akustische Warnung an einen Benutzer und/oder als elektronisches Warnsignal, beispielsweise an eine Steuereinheit der Anlage, ausgebildet sein. Anhand eines elektronischen Steuersignals kann die durch die Steuereinheit eine Gegenmaßnahme im Betrieb der Anlage eingeleitet werden.

Das erfindungsgemäße Überwachungsverfahren ist im Wesentlichen zweistufig aufgebaut. Zum einen wird anhand des zweiten und dritten Schritts erkannt, ob überhaupt eine bestimmungswidriger Betriebszustand vorliegt. Erst wenn ein bestimmungswidriger Betriebszustand erkannt wird, wird durch separates Erfassen und Auswerten von Zellen-Abweichungsparametern im Einzelnen analysiert, um so eine Defekt-Zelle zu erkennen und dadurch ein detailliertes Bild vom vorliegenden bestimmungswidrigen Betriebszustand zu gewinnen. Solange ein bestimmungsgemäßer Betriebszustand vorliegt, erfolgt im dritten Schritt lediglich ein Abgleich zwischen dem System-Abweichungsparameter und dem System-Schwellenwert. Dementsprechend wird im bestimmungsgemäßen Betriebszustand die Anzahl an durchzuführenden rechenintensiven Vergleichsoperationen minimiert. Der System-Abweichungsparameter kann beispielsweise als Summe von Zellen-Abweichungsparametern ausgebildet sein, die mit reduziertem Rechenaufwand ermittelbar ist. Erst bei einem erkannten bestimmungswidrigen Betriebszustand erfolgt mit den Zellen-Abweichungsparametern und den zugehörigen Zellen-Schwellenwerten eine gesteigerte Anzahl an Vergleichsoperationen. Das erfindungsgemäße Überwachungsverfahren ist folglich mit reduzierter Rechenleistung durchführbar. Das erfindungsgemäße Überwachungsverfahren, insbesondere der zweite, dritte und vierte Schritt, bieten infolgedessen Echtzeitfähigkeit für eine breite Spanne an Anlagen. Der Begriff der Echtzeitfähigkeit ist hierbei im jeweiligen Sinn der entsprechenden Anlage aufzufassen. Die Erfindung basiert unter anderem auf der Erkenntnis, dass der System-Abweichungsparameter im dritten Schritt als Entscheidungskriterium für das Durchführen des vierten Schritts zuverlässig einsetzbar ist. Das erfindungsgemäße Überwachungsverfahren bietet insgesamt eine gesteigerte Zuverlässigkeit bei reduziertem Rechenaufwand.

In einer Ausführungsform des beanspruchten Überwachungsverfahrens umfasst dieses einen fünften Schritt, in dem eine im vierten Schritt ermittelte Defekt-Zelle oder ein ermittelter Satz an Defekt-Zellen weiter genutzt werden. Im fünften Schritt wird die Defekt-Zelle bzw. der Satz an Defekt-Zellen erfasst und durch einen Musterabgleich ausgewertet. Mittels des Musterabgleichs wird im fünften Schritt eine Defektursache ermittelt. Dazu kann eine Datenbank mit einer Vielzahl an gespeicherten Vergleichsmustern unterschiedlicher Schadenszustände, also bestimmungswidriger Betriebszustände der Anlage, eingesetzt werden. Alternativ oder ergänzend kann der Musterabgleich auch mittels einer Künstlichen Intelligenz, beispielsweise eines Machine-Learning-Algorithmus durchgeführt werden, der vor und/oder während des Betriebs der Anlage trainiert wird. Beim Musterabgleich kann ebenso bei einer oder mehreren Defekt-Zellen berücksichtigt werden, in welchem Ausmaß, also um welchen Betrag, diese den zugehörigen Zellen-Schwellenwert betragsmäßig übersteigen. Als Defektursache kann beispielsweise ein defektes Gerät der Anlage, ein Wegfall einer Funktionalität eines solchen Geräts oder eine Fremdeinwirkung in die Anlage identifiziert werden. Da das beanspruchte Überwachungsverfahren mit reduzierter Rechenleistung durchführbar ist bzw. bei erhöhter eingesetzter Rechenleistung schnell durchführbar ist, ist eine solche Künstliche Intelligenz mitlaufend zum Betrieb der Anlage weiterentwickelbar, insbesondere weitertrainierbar. Das beanspruchte Überwachungsverfahren bietet hierdurch eine verbesserte Erkennung von bestimmungswidrigen Betriebszuständen der Anlage.

Weiter kann im beanspruchten Überwachungsverfahren der erste, zweite, dritte und/oder vierte Schritt mittels eines Autoencoders durchgeführt werden. Der System-Abweichungsparameter, der mittels des Autoencoders so ermittelt wird, kann ein sogenannter Total-Reconstruction-Error sein. Autoencoder sind in unterschiedlichen Konfigurationen verfügbar und weisen ein erhöhtes Maß an Zuverlässigkeit und Robustheit auf. Ferne sind Autoencoder leistungsfähig und erlauben die schnelle Verarbeitung einer Vielzahl an Eingangsdaten, also von umfangreichen Eingangssignal-Arrays. Das beanspruchte Überwachungsverfahren ist folglich in einfacher Weise umsetzbar und an unterschiedliche Anlagen anpassbar. Weiter erlaubt es der Autoencoder, eine Mehrzahl an Schritten des beanspruchten Überwachungsverfahren auszuführen. Ferner ist mit einem Autoencoder ein sogenanntes unbeaufsichtigtes Lernen, auch als unsupervised learning bezeichnet, möglich. Ein Datensatz zum Trainieren des Autoencoders kommt mit einer vereinfachten Sortierung, aus einem reduzierten Daten-Labeling, aus. Des Weiteren nutzt der Autoencoder in einem bestimmungsgemäßen Betriebszustand das Verhältnis von vorhandenen aktuellen Daten untereinander. Die Verwendung einer umfangreichen Datenbank ist somit in einem überwiegenden Anteil der Betriebsdauer entbehrlich.

Darüber hinaus kann zumindest einer der einstellbaren Zellen-Schwellenwerte im beanspruchten Überwachungsverfahren als fester Schwellenwert oder als gleitender Schwellenwert ausgebildet sein. Ein fester Schwellenwert bietet eine einfache Konfigurierbarkeit des Überwachungsverfahrens. Bei Zellen-Schwellenwerten, die auf einen Referenzwert normiert sind, ist das beanspruchte Überwachungsverfahren durch zumindest einen festen Schwellenwert in einfacher Weise konfigurierbar, beispielsweise bei einer Initialisierung. Ein gleitender Schwellenwert hingegen erlaubt es, beispielsweise einen Degradationseffekt auf ein Gerät, und damit auf eine entsprechende Zelle des Eingangssignal-Arrays, zu berücksichtigen. Beispielsweise kann der Zellen-Schwellenwert in Abhängigkeit von durchlaufenen Betriebsstunden eines Geräts entlang einer Funktion steigen oder sinken. Hierdurch werden unzutreffende Warnungen vermieden und/oder vorzeitig eintretende Degradationen von Geräten zuverlässig erkannt. Die Diagnosegenauigkeit des beanspruchten Überwachungsverfahrens wird so weiter gesteigert. Beispielsweise kann der Schwellenwert ein einstellbares Vielfaches einer Standardabweichung der entsprechenden Daten sein und/oder ein einstellbares Vielfaches eines mittleren Total-Reconstruction-Errors sein. Hierdurch können sowohl ein fester als auch ein gleitender Schwellenwert bereitgestellt werden. Ob der Schwellenwert gleitend oder fest ist, ist davon abhängig, ob die zugrundeliegenden Werte, beispielsweise die Standardabweichung oder der mittlere Total-Reconstruction-Error, im während des beanspruchten Überwachungsverfahren mitgeführt werden.

In einer weiteren Ausführungsform des beanspruchten Überwachungsverfahrens kann der zweite Schritt ein Interpolieren von Messwerten und/oder Steuerbefehlen umfassen, an das Eingangssignal-Array übergeben werden. Dem Eingangssignal-Array werden dadurch synchrone Messpunkte bereitgestellt, so dass jede Eingabe des Eingangssignal-Arrays einen Betriebszustand der Anlage in einem konkreten diskreten Zeitintervall beschreibt. Hierdurch ist eine erhöhte zeitliche Auflösung im beanspruchten Überwachungsverfahren erzielbar. Mehrdeutigkeiten oder Widersprüchlichkeiten in Eingaben in das Eingangssignal-Array bei transienten Betriebszuständen der Anlage, und damit unzutreffende Warnungen, werden so minimiert. Das Interpolieren kann als ein sogenanntes Upsampling ausgebildet sein. Infolgedessen ist das beanspruchte Überwachungsverfahren für zeitkritische Anwendungen geeignet, in denen beschleunigte Reaktionen auf den vorliegenden Betriebszustand geboten sind. Das beanspruchte Überwachungsverfahren weist dadurch eine erhöhte Zuverlässigkeit und Diagnosegenauigkeit auf. Alternativ kann im zweiten Schritt auch ein sogenanntes Downsampling erfolgen. Dadurch werden nur solche Messwerte und/oder Steuerbefehle an das Eingangssignal-Array übergeben, die bereits in einem diskreten Zeitintervall liegen. Hierdurch wird die für das beanspruchte Überwachungsverfahren erforderliche Rechenleitung reduziert.

Ferner kann das Eingangssignal-Array und/oder das Überwachungsdaten-Array Zellen umfassen, in denen Messwerte und/oder Steuerbefehle während des beanspruchten Überwachungsverfahren gespeichert werden, die einem einstellbaren Relevanz-Kriterium genügen. Das Relevanzkriterium kann beispielsweise eine Angabe darüber sein, bei wie vielen bekannten Defekten bei der entsprechenden Zelle ein Überschreiten des Zellen-Schwellenwerts zu erwarten ist. Eine Zelle, die bei einer minimalen Anzahl an bekannten Defekten, insbesondere bei keinem bekannten Defekt, den Zellen-Schwellwert betragsmäßig übersteigt, kann aus dem Eingangssignal-Array entfernt werden. Das beanspruchte Überwachungsverfahren kann sich folglich mit fortschreitender Betriebsdauer selbsttätig weiter optimieren, und somit beschleunigen. Dies erlaubt unter anderem ein einfaches Modifizieren der Anlage, ohne durch die durchgeführten Modifikationen die Leistungsfähigkeit des beanspruchten Überwachungsverfahrens zu belasten. Das Relevanz-Kriterium kann insbesondere zu einem sogenannten Pruning, beispielsweise des Eingangssignal-Arrays, eingesetzt werden.

Die zugrundeliegende Aufgabenstellung wird gleichermaßen durch ein weiteres erfindungsgemäßes Überwachungsverfahren gelöst. Das Überwachungsverfahren dient dazu, eine Anlage zu überwachen, die eine Mehrzahl an Geräten aufweist, die zu einem bestimmungsgemäßen Betrieb der Anlage zusammenwirken. Die Geräte können beispielsweise Sensoren, Steuereinheiten, Regelungseinheiten oder Kombinationen hieraus sein. Die Geräte sind dazu ausgebildet, Knoten in einem neuronalen Netz zu bilden, das das Zusammenwirken der Geräte in der Anlage abbildet. Das neuronale Netz umfasst Nullkorrelationen und/oder Betriebskorrelationen. Die Geräte können physikalisch separate Geräte sein, oder unterschiedliche Funktionalitäten in physikalisch einzelnen Geräten sein. Das Überwachungsverfahren umfasst einen ersten Schritt, in dem ein trainiertes neuronales Netz bereitgestellt wird, das eine Mehrzahl an Kanten aufweist, die jeweils einer Nullkorrelation oder einer Betriebskorrelation zwischen zwei Knoten des neuronalen Netzes entsprechen. Die Knoten werden hierbei durch Geräte der Anlage gebildet. Unter einer Nullkorrelation ist eine Korrelation mit einem Korrelationswert zu verstehen, die unterhalb eines einstellbaren Korrelationsschwellenwerts liegt. Eine sog. Nullkorrelation beschreibt einen schwachen, vernachlässigbaren oder nicht-existenten Zusammenhang zwischen zwei Knoten. Der Begriff Nullkorrelation ist im Sinne der bisher unveröffentlichten deutschen Patentanmeldung mit dem amtlichen Aktenzeichen 10 2022 200 694.1 aufzufassen. Der Offenbarungsgehalt der Akte 10 2022 200 694.1 wird durch Verweisung in die vorliegende Anmeldung miteinbezogen. Die Nullkorrelationen weisen im bereitgestellten trainierten neuronalen Netz Korrelationswerte auf, die unterhalb des einstellbaren Korrelationsschwellenwerts liegen. Die entsprechenden Korrelationswerte werden beim Trainieren des neuronalen Netzes ermittelt. Die Nullkorrelationen beschreiben somit Paare von Geräten, zwischen denen in einem bestimmungsgemäßen Betriebszustand kein erkennbarer Zusammenhang zu erwarten ist. Unter Betriebskorrelationen sind Korrelationen zu verstehen, bei denen zwischen zwei Knoten ein hinreichend starker Zusammenhang besteht. Eine Betriebskorrelation ist folglich das Gegenteil bzw. das Gegenstück zu einer Nullkorrelation. Der Begriff Betriebskorrelation ist korrespondierend zum Begriff Nullkorrelation gemäß der Akte 10 2022 200 694.1 aufzufassen.

Des Weiteren umfasst das Überwachungsverfahren einen zweiten Schritt, in dem die Anlage in einem aktiven Betriebszustand bereitgestellt wird und Messwerte und/oder Steuerbefehle erfasst werden, die im aktiven Betriebszustand auftreten. Die Messwerte können erfasste physikalische Größen sein oder Zustandsangaben über das jeweilige Gerät sein. Die erfassten Messwerte und/oder Steuerbefehle korrespondieren jeweils mit zumindest einem Knoten und/oder einer Kante des neuronalen Netzes. Die erfassten Messwerte und/oder Steuerbefehle sind dazu geeignet, als Eingabe für das neuronale Netz zu dienen.

In einem dritten Schritt wird ein Netz-Abweichungsparameter ermittelt, der Korrelationswerte einer Mehrzahl an Kanten des neuronalen Netzes kombiniert. Dazu werden Korrelationswerte für mehrere Kanten basierend auf den im zweiten Schritt erfassten Messwerten und/oder Steuerbefehlen ermittelt, die beispielsweise betragsmäßig in einer Summe zusammengefasst werden. Die Korrelationswerte können für Kanten von Nullkorrelationen und/oder von Betriebskorrelationen erfasst werden um den Netz-Abweichungsparameter zu ermitteln. Der Netz-Abweichungsparameter stellt somit eine komprimierte Größe dar, die einen Gesamtstatus der Anlage abbildet. Darauf folgt ein vierter Schritt, in dem ein bestimmungswidriger Betriebszustand der Anlage erkannt wird, wenn der Netz-Abweichungsparameter einen einstellbaren Netz-Schwellenwert betragsmäßig übersteigt. Der Netz-Schwellenwert ist durch einen Benutzer oder einen Algorithmus vorgebbar und stellt ein Maß dar, das eine Abweichung von einem angestrebten Soll-Zustand der Anlage abbildet. Ein Übersteigen bzw. Überschreiten des Netz-Schwellenwerts ist folglich dadurch hervorrufbar, dass Korrelationswerte von Kanten, die bestimmungsgemäß Nullkorrelationen sind, derart ansteigen, dass diese einzeln oder kombiniert nicht mehr zutreffend als Nullkorrelationen kategorisierbar sind. Das erfindungsgemäße Überwachungsverfahren erkennt somit das Vorhandensein von Schein-Korrelationen, wo in einem bestimmungsgemäßen Betriebszustand Nullkorrelationen vorliegen bzw. vorzuliegen haben. Alternativ oder ergänzend können auch Betriebskorrelationen Abweichungen aufweisen, die kombiniert zu einem Überschreiten bzw. Übersteigen des Netz-Schwellenwerts führen. Das erfindungsgemäße Überwachungsverfahren beruht unter anderem auf der überraschenden Erkenntnis, dass bestimmungswidrige Betriebszustände in einfacher und zuverlässiger Weise anhand von Nullkorrelationen erkennbar sind, die ansonsten für den Betrieb keine nähere Bedeutung haben. Nähere Kenntnisse über die Nullkorrelationen sind entbehrlich, da diese frei von zugrundeliegenden physikalischen Zusammenhängen sind. Ferner sind Kanten mit Nullkorrelationen in erhöhter Anzahl im Wesentlichen selbsttätig erzeugbar. Gleichermaßen kann auch ein Abweichen von Betriebskorrelationen in einfacher Weise erkannt werden und ein bestimmungswidriger Betriebszustand erkannt werden. Wenn der bestimmungswidrige Betriebszustand erkannt wird, wird eine Warnung ausgegeben. Die Warnung kann eine visuelle oder akustische Warnung an einen Benutzer und/oder ein elektronisches Warnsignal, beispielsweise an eine Steuereinheit der Anlage, sein. Anhand eines elektronischen Steuersignals kann die durch die Steuereinheit eine Gegenmaßnahme im Betrieb der Anlage eingeleitet werden.

Der Netz-Abweichungsparameter ist mit reduziertem Rechenaufwand bereitstellbar und über ein Minimum an Vergleichsoperationen auf das betragsmäßige Überschreiten des Netz-Schwellenwerts überprüfbar. Dementsprechend ist das erfindungsgemäße Überwachungsverfahren mit reduziertem Rechenaufwand bzw. mit erhöhter Geschwindigkeit durchführbar. Durch den ersten, zweiten, dritten und vierten Schritt wird somit die Echtzeitfähigkeit des erfindungsgemäßen Überwachungsverfahrens gesteigert. Der Begriff Echtzeitfähigkeit ist hierbei im Lichte der konkreten Anwendung, also der Anlage, die mit dem Überwachungsverfahren überwacht wird, aufzufassen. Gleichzeitig erlaubt das Überwachungsverfahren eine zuverlässige Diagnose eines vorliegenden bestimmungswidrigen Betriebszustands.

Gemäß einer Ausführungsform des beanspruchten Überwachungsverfahrens können der zweite, dritte und/oder vierte Schritt separat für Nullkorrelationen und Betriebskorrelationen durchgeführt werden. Insbesondere sind der zweite, dritte und/oder vierte Schritt für die Nullkorrelationen mit einer einstellbaren ersten Frequenz durchführbar. Weiter können der zweite, dritte und/oder vierte Schritt für die Betriebskorrelationen mit einer einstellbaren zweiten Frequenz durchgeführt werden. Die erste bzw. zweite Frequenz sind durch einen Benutzer oder einen Algorithmus vorgebbar. Des Weiteren kann die erste Frequenz geringer sein als die zweite Frequenz. Das beanspruchte Überwachungsverfahren ist daher an unterschiedliche Anwendungen anpassbar, in denen ein bestimmungswidriger Betriebszustand erwartungsgemäß eher über die Betriebskorrelationen bzw. die Nullkorrelationen erkennbar ist. Insgesamt weist das beanspruchte Überwachungsverfahren ein breites Einsatzspektrum auf. Weiter alternativ können der zweite, dritter und/oder vierte Schritt in einem ersten Durchgang für die Betriebskorrelationen ausgeführt werden. Wenn im ersten Durchgang ein bestimmungswidriger Betriebszustand erkannt wird, können der zweite, dritte und/oder vierte Schritt für die Nullkorrelationen durchgeführt werden. Hierbei werden im ersten und zweiten Durchgang separate Netz-Abweichungsparametern ermittelt und mit jeweils zugehörigen einstellbaren Netz-Schwellenwerten abgeglichen. Das beanspruchte Überwachungsverfahren kann so mit reduzierter Rechenleistung umgesetzt werden.

In einer weiteren Ausführungsform des beanspruchten Überwachungsverfahrens umfasst dieses einen fünften Schritt, in dem eine Kante des neuronalen Netzes, die in einem bestimmungsgemäßen Betriebszustand der Anlage einer Nullkorrelation entspricht, als Defekt-Kante erkannt. Die Kante wird als Defekt-Kante erkannt, wenn deren Korrelationswert den zugehörigen Korrelationsschwellenwert betragsmäßig übersteigt. Der fünfte Schritt wird durchgeführt, wenn im vierten Schritt der bestimmungswidrige Betriebszustand der Anlage erkannt ist. Das betragsmäßige Übersteigen des Korrelationsschwellenwerts ist durch einen separaten Vergleich der Korrelationswerte mit dem korrespondierenden Korrelationsschwellenwert durchgeführt werden. Dadurch werden rechenintensive Vergleichsoperationen lediglich dann durchgeführt, wenn anhand des überschrittenen Netz-Schwellenwerts zu erwarten ist, dass zumindest ein Korrelationsschwellenwert überschritten ist und häufige ergebnislose Durchgänge von rechenintensiven Operationen vermeidbar sind. Das erfindungsgemäße Überwachungsverfahren bietet folglich einen rationellen Umgang mit der eingesetzten Rechenleistung. Gleichzeitig bietet das beanspruchte Überwachungsverfahren eine zielgerichtete Diagnose.

Darüber hinaus kann der Korrelationsschwellenwert zumindest einer Kante im neuronalen Netz als fester Schwellenwert oder als gleitender Schwellenwert ausgebildet sein. Ein fester Schwellenwert bietet eine einfache Konfigurierbarkeit des Überwachungsverfahrens. Bei Korrelationsschwellenwerten, die auf einen Referenzwert normiert sind, ist das beanspruchte Überwachungsverfahren durch zumindest einen festen Schwellenwert in einfacher Weise konfigurierbar, beispielsweise bei einer Initialisierung. Ein gleitender Schwellenwert hingegen erlaubt es, beispielsweise einen Degradationseffekt auf ein Gerät, und damit auf eine entsprechende Kante des neuronalen Netzes zu berücksichtigen. Der gleitende Schwellenwert kann beispielsweise durch einen veränderlichen Total-Reconstruction-Error gebildet sein. Beispielsweise kann der Korrelationsschwellenwert in Abhängigkeit von durchlaufenen Betriebsstunden eines Geräts entlang einer Funktion steigen oder sinken. Hierdurch werden unzutreffende Warnungen vermieden und/oder vorzeitig eintretende Degradationen von Geräten zuverlässig erkannt. Die Diagnosegenauigkeit des beanspruchten Überwachungsverfahrens wird so weiter gesteigert.

Ferner kann das beanspruchte Überwachungsverfahren einen sechsten Schritt aufweisen, in dem basierend auf der, beispielsweise im fünften Schritt, erkannten Defekt-Kante ein Musterabgleich durchgeführt werden. Im sechsten Schritt wird die Defekt-Kante erfasst und durch einen Musterabgleich ausgewertet. Mittels des Musterabgleichs wird im sechsten Schritt eine Defektursache ermittelt. Dazu kann eine Datenbank mit einer Vielzahl an gespeicherten Vergleichsmustern unterschiedlicher Schadenszustände, also bestimmungswidriger Betriebszustände der Anlage, eingesetzt werden. Alternativ oder ergänzend kann der Musterabgleich auch mittels einer Künstlichen Intelligenz, beispielsweise eines Machine-Learning-Algorithmus durchgeführt werden, der vor und/oder während des Betriebs der Anlage trainiert wird. Beim Musterabgleich kann ebenso bei einer oder mehreren Defekt-Kanten berücksichtigt werden, in welchem Ausmaß, also um welchen Betrag, diese den zugehörigen Korrelationsschwellenwert betragsmäßig übersteigen. Als Defektursache kann beispielsweise ein defektes Gerät der Anlage, ein Wegfall einer Funktionalität eines solchen Geräts oder eine Fremdeinwirkung in die Anlage identifiziert werden. Da das beanspruchte Überwachungsverfahren mit reduzierter Rechenleistung durchführbar ist bzw. bei erhöhter eingesetzter Rechenleistung schnell durchführbar ist, ist eine solche Künstliche Intelligenz mitlaufend zum Betrieb der Anlage weiterentwickelbar, insbesondere weitertrainierbar. Das beanspruchte Überwachungsverfahren bietet hierdurch eine verbesserte Erkennung von bestimmungswidrigen Betriebszuständen der Anlage.

Die beschriebene Aufgabenstellung wird gleichermaßen durch ein erfindungsgemäßes Computerprogrammprodukt gelöst. Das Computerprogrammprodukt ist dazu ausgebildet, Messwerte und/oder Steuersignale von Geräten in einer Anlage zu empfangen, zumindest vorübergehend zu speichern und zu verarbeiten. Das Computerprogrammprodukt ist auch dazu ausgebildet, eine Warnung an einen Benutzer und/oder ein elektronisches Warnsignal auszugeben. Das Computerprogrammprodukt dient zu einem Überwachen der Anlage, zu der die Geräte gehören und zu deren Betrieb sie zusammenwirken. Die Messwerte und/oder Steuerbefehle werden im Rahmen eines Überwachungsverfahrens verarbeitet, das mitlaufend, also im Wesentlichen parallel, insbesondere in Echtzeit, zum Betrieb der Anlage ablaufen kann. Erfindungsgemäß ist das Computerprogrammprodukt dazu ausgebildet, ein Überwachungsverfahren nach zumindest einem der oben skizzierten Ausführungsformen durchzuführen. Das Computerprogrammprodukt kann monolithisch, also zum Betrieb auf einer einzigen Hardwareplattform, ausgebildet sein. Alternativ kann das Computerprogrammprodukt modular ausgebildet sein, also Teilprogramme umfassen, die auf separaten Hardwareplattformen, beispielsweise einer Computer-Cloud, ausführbar sind und über eine kommunikative Datenverbindung zusammenwirken, um das jeweilige Überwachungsverfahren auszuführen. Das erfindungsgemäße Computerprogrammprodukt erlaubt es, die erfindungsgemäßen Überwachungsverfahren in einfacher Weise zu implementierten.

Ebenso wird die zugrundeliegende Aufgabe durch eine erfindungsgemäße Überwachungseinheit für eine Anlage gelöst. Die Überwachungseinheit ist dazu ausgebildet, einen Betrieb der Anlage, mit der sie zumindest funktionell gekoppelt ist, zu überwachen und eine Warnung auszugeben. Die zu überwachende Anlage umfasst eine Mehrzahl an Geräten, die zu einem Betrieb der Anlage zusammenwirken und zu denen Messwerte und/oder Steuerbefehle erfassbar sind. Das Erfassen der Messwerte und/oder Steuerbefehle, das auch ein Empfangen umfasst, erfolgt hierbei durch die Überwachungseinheit. Ebenso ist die Überwachungseinheit dazu ausgebildet, die erfassten Messwerte und/oder Steuerbefehle zu verarbeiten. Erfindungsgemäß ist die Überwachungseinheit dazu ausgebildet, die Messwerte und/oder Steuerbefehle mittels eines Computerprogrammprodukts zu verarbeiten, mit dem die Überwachungseinheit ausgestattet ist. Das Computerprogrammprodukt ist erfindungsgemäß nach einer der oben dargestellten Ausführungsformen ausgebildet.

Die eingangs dargelegte Aufgabenstellung wird ferner durch eine erfindungsgemäße Gasanalysevorrichtung gelöst. Die Gasanalysevorrichtung umfasst eine Mehrzahl an Geräten, die zusammenwirken und eine Stoffprobe aufzubereiten und zu vermessen. Insbesondere kann die Gasanalysevorrichtung dazu ausgebildet sein, eine Zusammensetzung der Stoffprobe zumindest qualitativ zu ermitteln. Die Gasanalysevorrichtung ist mit einer Überwachungseinheit versehen, die mit einer Mehrzahl an Geräten gekoppelt ist. Die Überwachungseinheit ist dazu ausgebildet, einen bestimmungswidrigen Betriebszustand der Gasanalysevorrichtung zu erkennen. Erfindungsgemäß ist die Überwachungseinheit gemäß einer der oben dargelegten Ausführungsformen ausgebildet.

Weiter wird die skizzierte Aufgabe durch eine erfindungsgemäße Verwendung einer Künstlichen Intelligenz gelöst, die zu einem Überwachen einer Gasanalysevorrichtung ausgebildet ist. Die Künstliche Intelligenz ist dazu geeignet, Messwerte und/oder Steuerbefehle von Geräten der Gasanalysevorrichtung mittelbar oder unmittelbar zu empfangen und zu verarbeiten. Die Verwendung der Künstlichen Intelligenz umfasst zumindest das Ausgeben einer Warnung, die beispielsweise eine visuelle oder akustische Warnung an einen Benutzer und/oder ein elektronisches Warnsignal sein kann. Zum Überwachen der Gasanalysevorrichtung ist die Künstliche Intelligenz dazu ausgebildet, eine Überwachungsverfahren durchzuführen. Erfindungsgemäß kann das Überwachungsverfahren gemäß einer der oben beschriebenen Ausführungsformen ausgebildet sein. Das Verwenden der Künstlichen Intelligenz kann insbesondere das Ablaufen eines entsprechend ausgebildeten Computerprogrammprodukts umfassen, wie oben beispielsweise dargelegt.

Die Erfindung wird im Folgenden anhand einzelner Ausführungsformen in Figuren näher erläutert. Die Figuren sind insoweit in gegenseitiger Ergänzung zu lesen, dass gleiche Bezugszeichen in unterschiedlichen Figuren die gleiche technische Bedeutung haben. Die Merkmale der einzelnen Ausführungsformen sind untereinander auch kombinierbar. Ferner sind die in den Figuren gezeigten Ausführungsformen mit den oben skizzierten Merkmalen kombinierbar. Es zeigen im Einzelnen:
- FIG 1: schematisch einen Ablauf einer ersten Ausführungsform des beanspruchten Überwachungsverfahrens;
- FIG 2: schematisch einen Ablauf einer zweiten Ausführungsform des beanspruchten Überwachungsverfahrens.

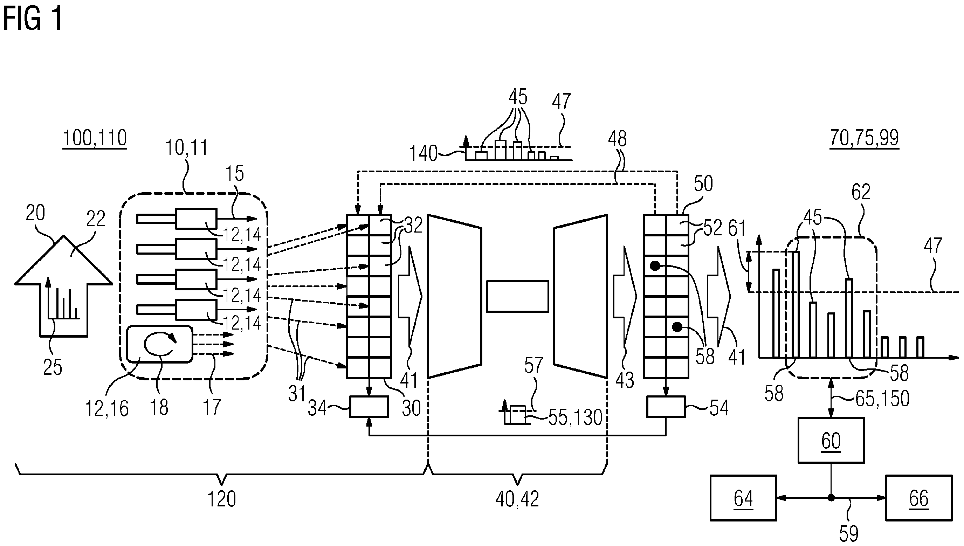

Eine erste Ausführungsform des beanspruchten Überwachungsverfahrens 100 ist schematisch in FIG 1 dargestellt. Das Überwachungsverfahren 100 dient dazu, eine Anlage 10 zu überwachten, mit der ein Anlagenprozess 20 durchgeführt wird. Die Anlage 10 ist als Gasanalysevorrichtung 11 ausgebildet und der darauf durchgeführte Anlagenprozess 20 umfasst, dass eine Zusammensetzung 25 einer Stoffprobe 22 ermittelt wird. Dazu ist die Anlage 10 mit einer Mehrzahl an Geräten 12 ausgestattet, die als Sensoren 14 oder als Steuereinheit 14 ausgebildet sind. Die Steuereinheit 16 ist dazu ausgebildet, durch ein ablaufendes Steuerprogramm 18 auf den Anlagenprozess 20 einzuwirken. Die Sensoren 14 erfassen für die Anlagenprozess 20 relevante physikalische Größen. Die Sensoren 14 sind dazu ausgebildet, Messwerte 15 auszugeben und die Steuereinheit 14 dazu, Steuerbefehle 17 auszugeben. Die in FIG 1 gezeigte erste Ausführungsform geht davon aus, dass ein erster Schritt 110 abgeschlossen ist, in dem ein Eingangssignal-Array 30 mit einer Mehrzahl an Zellen 32 bereitgestellt ist. Ebenso wird im ersten Schritt 110 ein Überwachungsdaten-Array 50 bereitgestellt, das eine Mehrzahl an Zellen 52 aufweist. Die Zellen 52 des Überwachungsdaten-Arrays 50 und die Zellen 32 des Eingangssignal-Arrays 32 korrespondieren miteinander, so dass das Eingangsdaten-Array 32 und das Überwachungsdaten-Array 52 in puncto Aufbau identisch sind.

In der ersten Ausführungsform des Überwachungsverfahrens 100 erfolgt ein zweiter Schritt 120, in dem die Anlage 10 in einem aktiven Betriebszustand bereitgestellt wird, in dem durch die Sensoren 14 Messwerte 15 und/oder durch die Steuereinheit 16 Steuerbefehle 17 für das Eingangssignal-Array 30 bereitgestellt werden. Das Bereitstellen 31 der Messwerte 15 bzw. der Steuerbefehle 17 erfolgt durch ein separates Einspeichern in einer Zelle 32 des Eingangsdaten-Arrays 30. Weiter erfolgt ein Bereitstellen 41 des Eingangssignal-Arrays 30 an eine Weiterverarbeitungseinheit 40, die als Autoencoder 42 ausgebildet ist. Mittels der Weiterverarbeitungseinheit 40 wird ein dritter Schritt 130 des Überwachungsverfahrens 100 durchgeführt.

Mittels der Weiterverarbeitungseinheit 40 werden basierend auf dem Eingangssignal-Array 30 Inhalte, also Werte, für Zellen 52 des Überwachungsdaten-Arrays 50 ermittelt. Anhand dessen wiederum wird ein Vergleichswert 54 des Überwachungsdaten-Arrays 50 ermittelt, der die Inhalte dessen Zellen 52 kombiniert. Ebenso wird ein Vergleichswert 34 des Eingangssignal-Arrays 30 ermittelt, das ebenso die Inhalte dessen Zellen 32 kombiniert. Die Vergleichswerte 34, 54 können beispielsweise jeweils als Summe ausgebildet sein. Weiter wird durch Abgleichen der Vergleichswerte 34, 54 des Eingangssignal-Arrays 30 und des Überwachungsdaten-Arrays 50 ein System-Abweichungsparameter 55 ermittelt. Der System-Abweichungsparameter 55 ist ein Total-Reconstruction-Error und stellt in kompakter Form einen Gesamtzustand der Anlage 10 dar. Der System-Abweichungsparameter 55 wird weiter im dritten Schritt 130 mit einem System-Schwellenwert 57 verglichen. Wenn der System-Abweichungsparameter 55 den System-Schwellenwert 57 betragsmäßig übersteigt, wird ein bestimmungswidriger Betriebszustand der Anlage 10 erkannt. Der im dritten Schritt 130 erkannte bestimmungswidrige Betriebszustand ist unspezifisch, ist also nicht dazu geeignet, eine zugrundeliegende Defektursache zu anzugeben. Wenn im dritten Schritt 130 der bestimmungswidrige Betriebszustand der Anlage 10 erkannt ist, folgt im beanspruchten Überwachungsverfahren 100 ein vierter Schritt 140.

Im vierten Schritt 140 werden die Inhalte von Zellen 52 des Überwachungsdaten-Arrays 50 mit den Inhalten von Zellen 32 des Eingangssignal-Arrays 30 separat, also individuell, verglichen. Die als Autoencoder 42 ausgebildete Weiterverarbeitungseinheit 40 bildet in einem bestimmungsgemäßen Betriebszustand der Anlage 10 die Inhalte der Zellen 32 des Eingangssignal-Arrays 30 im Wesentlichen identisch als Inhalte in die Zellen 52 des Überwachungsdaten-Arrays 50 ab. Das Abbilden 43 erfolgt durch ein zellenweises Einspeichern von Inhalten. Dementsprechend wird im vierten Schritt 140 separat für eine Mehrzahl an Zellen 32 des Eingangssignal-Arrays 30 und korrespondierenden Zellen 52 des Überwachungsdaten-Arrays 50 ein Zellen-Abweichungsparameter 45 ermittelt. Der Zellen-Abweichungsparameter 45 kann beispielsweise durch eine Differenz zwischen den zugehörigen Zellen 32, 53 ermittelt werden. Die so ermittelten Zellen-Abweichungsparameter 45 werden jeweils mit einem einstellbaren Zellen-Schwellenwert 47 verglichen. Ein solches Vergleichen ist in FIG 1 durch die Pfeile 48 versinnbildlicht. Wenn ein Zellen-Abweichungsparameter 45 den zugehörigen einstellbaren Zellen-Schwellenwert 47 betragsmäßig überteigt, wird die zugehörige Zelle 32, 52 des Eingangssignal-Arrays 30 bzw. des Überwachungsdaten-Arrays 50 als Defekt-Zelle 58 erkannt. Dazu werden die Defekt-Zellen 58 entsprechend markiert, beispielsweise mit einem sogenannten Signatur-Bit.

Ferner erfolgt im Überwachungsverfahren 100 ein fünfter Schritt 150, für den die Inhalte der Zellen 52 des Überwachungsdaten-Arrays 50 zum Abgleich mit einer Datenbank 60 bereitgestellt werden. Das Bereitstellen für den fünften Schritt 150 ist in FIG 1 durch einen Pfeil 41 dargestellt. Im fünften Schritt 150 wird für zumindest eine Defekt-Zelle 58 eine Überschreitungsweite 61 ermittelt, um die der Inhalt der Defekt-Zelle 58 den zugehörigen Zellen-Abweichungsparameter 45 betragsmäßig übersteigt. Weiter wird erfasst, bei welchen Zellen 32, 52 des Überwachungsdaten-Arrays 50 bzw. des Eingangssignal-Arrays 30 der entsprechende Zellen-Schwellenwert 47 betragsmäßig überschritten wird. Daraus ergibt sich, beispielsweise im Zusammenhang mit Zellen-Abweichungsparametern 45, die den zugehörigen Zellen-Schwellenwert 47 betragsmäßig unterschreiten, ein Muster 62, das für eine zugrundeliegende Defektursache charakteristisch ist. Das Muster 62 wird im fünften Schritt 150 durch einen Musterabgleich 65 mittels der Datenbank 60 erkannt. In der Datenbank 60 ist eine Mehrzahl an Mustern 62 gespeichert, die als Vergleichsmuster dienen. Wird ein Muster 62 im fünften Schritt 150 erkannt, wird eine entsprechende Warnung 59 ausgegeben. Die Warnung 59 kann das Vorliegen des bestimmungswidrigen Betriebszustands der Anlage 10 anzeigen, eine Angabe über ein zu mindestens einer Defekt-Zelle 58 zugehöriges defektes Gerät 12, und/oder eine Angabe über die erkannte Defektursache. Die Warnung 59 kann an eine Anzeigeeinheit 64 geleitet werden, die dazu ausgebildet ist, die Warnung 59 beispielsweise als visuelle oder akustische Warnung an einen Benutzer auszugeben. Alternativ oder ergänzend kann die Warnung 59 als elektronisches Warnsignal ausgebildet sein, das an eine Kommunikationsschnittstelle 66 ausgegeben wird, die mit einer nicht näher gezeigten übergeordneten Steuereinheit verbunden sein kann. Das Überwachungsverfahren 100 in FIG 1 wird durch ein Computerprogrammprodukt 70 umgesetzt, das auf einer Überwachungseinheit 75 ausgeführt wird. Das Computerprogrammprodukt 70 bildet eine Künstliche Intelligenz 99, die dazu eingesetzt wird, die als Gaschromatograph 11 ausgebildete Anlage 10 zu überwachen.

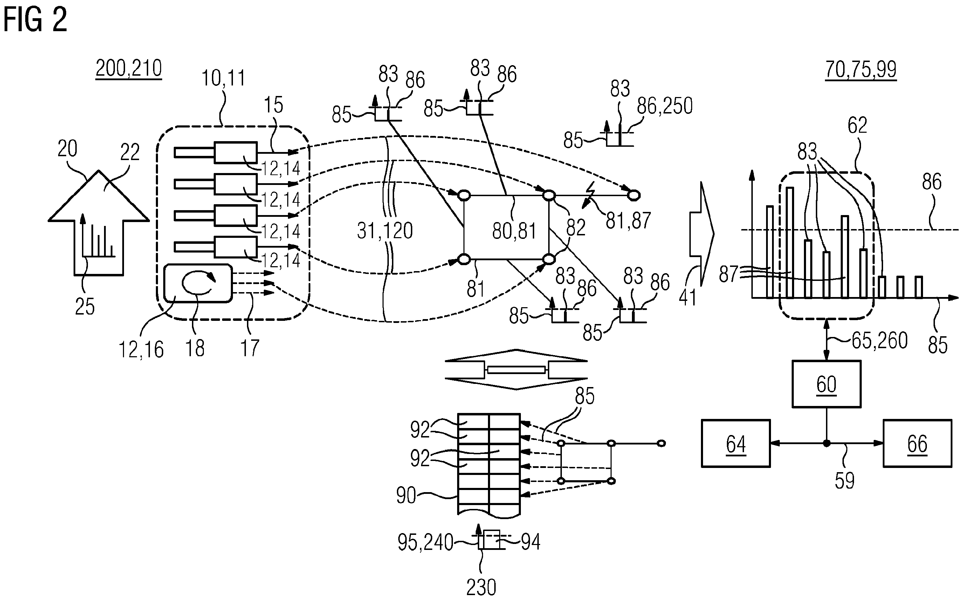

Eine zweite Ausführungsform des beanspruchten Überwachungsverfahrens 200 ist schematisch in FIG 2 abgebildet. Das Überwachungsverfahren 200 dient dazu, eine Anlage 10 zu überwachten, mit der ein Anlagenprozess 20 durchgeführt wird. Die Anlage 10 ist als Gasanalysevorrichtung 11 ausgebildet und der darauf durchgeführte Anlagenprozess 20 umfasst, dass eine Zusammensetzung 25 einer Stoffprobe 22 ermittelt wird. Dazu ist die Anlage 10 mit einer Mehrzahl an Geräten 12 ausgestattet, die als Sensoren 14 oder als Steuereinheit 14 ausgebildet sind. Die Steuereinheit 16 ist dazu ausgebildet, durch ein ablaufendes Steuerprogramm 18 auf den Anlagenprozess 20 einzuwirken. Die Sensoren 14 erfassen für die Anlagenprozess 20 relevante physikalische Größen. Die Sensoren 14 sind dazu ausgebildet, Messwerte 15 auszugeben und die Steuereinheit 14 dazu, Steuerbefehle 17 auszugeben.

Die in FIG 2 gezeigte zweite Ausführungsform geht davon aus, dass ein erster Schritt 210 abgeschlossen ist, in dem ein neuronales Netz 80 bereitgestellt ist, das für einen Einsatz im Überwachungsverfahren 200 trainiert ist. Das neuronale Netz 80 umfasst eine Mehrzahl an Knoten 82, die über Kanten 81 miteinander verbunden sind. Jeder Kante 81 ist ein Korrelationswert 83 zugeordnet, der wiedergibt, wie stark eine Änderung an einem Knoten 82, also einem dort vorliegenden Messwert 15 oder Steuerbefehl 17, einem über die entsprechende Kante 81 verbundenen Knoten 82 folgt. Die Korrelationsschwellenwerte 83 sind beispielsweise durch eine Benutzereingabe, eine Wertetabelle oder einen Algorithmus, insbesondere eine Künstliche Intelligenz, einstellbar. Die Kanten 81 im neuronalen Netz 80 nach FIG 2 sind in einem bestimmungsgemäßen Betriebszustand der Anlage 10 als Null-Korrelationen 85 ausgebildet. Dementsprechend liegen die Korrelationswerte 83 der entsprechenden Kanten 81 betragsmäßig unter einem einstellbaren Korrelationsschwellenwert 86, die im Wesentlichen ein Minimum an Wechselwirkung zwischen den entsprechenden Knoten 82 beschreibt. Die Null-Korrelationen 85 im neuronalen Netz 80 beschreiben somit Messwerte 15 bzw. Steuerbefehle 17, zwischen denen im bestimmungsgemäßen Zustand noch nicht einmal ein Scheinzusammenhang besteht. Die Kanten 81, die Nullkorrelationen 85 entsprechen, werden im ersten Schritt 210 durch Trainieren des neuronalen Netzes 80 ermittelt.

Das Überwachungsverfahren 200 umfasst einen zweiten Schritt 220, der auf dem abgeschlossenen ersten Schritt 210 aufbaut. Im zweiten Schritt 220 wird die Anlage 10 in einem aktiven Betriebszustand bereitgestellt, in dem der Anlagenprozess 20 durchgeführt wird und die Geräte 12, also die Sensoren 14 und die Steuereinheit 16, Messwerte 15 bzw. Steuerbefehle 17 erzeugen und bereitstellen. Das Bereitstellen 31 ist in FIG 2 durch Pfeile versinnbildlicht. Die Messwerte 15 bzw. die Steuerbefehle 17 korrespondieren jeweils mit einem Knoten 82 und/oder einer Kante 81 des neuronalen Netzes 80 und werden als Eingabewerte in das neuronale Netz 80 eingespeist. Es folgt ein dritter Schritt 230, in dem ausgehend von den im zweiten Schritt 220 bereitgestellten Messwerten 15 bzw. Steuerbefehlen 17 die an den jeweiligen Kanten 81 vorliegenden Korrelationswerte 83 ermittelt, also berechnet, werden. Die vorliegenden Korrelationswerte 83 werden beispielsweise in Zellen 92 einer Tabelle 90 gespeichert und ausgehend hiervon ein Netzabweichungsparameter 94 ermittelt. Der Netz-Abweichungsparameter 94 kann beispielsweise als Summe von Beträgen der Korrelationswerte 83 gebildet werden. Der Netz-Abweichungsparameter 94 ist ein Maß für das Vorliegen eines unspezifizierten bestimmungswidrigen Betriebszustand der Anlage 10. Weiter umfasst das Überwachungsverfahren 200, dass ein vierter Schritt 240 durchgeführt wird. Im vierten Schritt 240 wird der im dritten Schritt 230 ermittelte Netz-Abweichungsparameter 94 mit einem Netz-Schwellenwert 95 verglichen. In einem bestimmungsgemäßen Betriebszustand der Anlage 10 unterschreitet der Netz-Abweichungsparameter 94 den Netz-Schwellenparameter 95 betragsmäßig. Der Netz-Schwellenwert 95 kann als fester Schwellenwert oder als gleitender Schwellenwert ausgebildet sein, der beispielsweise unter Berücksichtigung von absolvierten Betriebsstunden der Anlage 10 ansteigen kann. Bei erkanntem Vorliegen eines bestimmungswidrigen Betriebszustands kann eine Warnung ausgegeben werden.

Ferner gehört ein fünfter Schritt 250 zum Überwachungsverfahren 200 nach FIG 2, in dem die Korrelationswerte 83 der einzelnen Kanten 81 mit den zugehörigen Korrelations-Schwellenwerten 86 verglichen werden. Wenn der Korrelationswert 83 einer Kante 81 den zugehörigen Korrelationsschwellenwert 86 betragsmäßig übersteigt, wird die zugehörige Kante 81 als Defekt-Kante 87 erkannt. Die Defekt-Kante 87 erlaubt einen Rückschluss darauf, dass der Messwert 15 bzw. Steuerbefehl 17 an einem der Knoten 82, die durch die Kante 81 verbunden sind, durch einen bestimmungswidrigen Betriebszustand der Anlage 10 entstanden ist. Dementsprechend ist diagnostizierbar, dass ein Gerät 12, das zum entsprechenden Knoten 82 gehört, defekt ist oder zumindest als defekt zu erwarten ist.

Darüber hinaus umfasst das Überwachungsverfahren 200 einen sechsten Schritt 260, in dem zumindest die Korrelationswerte 83 zu einer weiteren Auswertung bereitgestellt werden. Das Bereitstellen 41 ist durch einen Pfeil in FIG 2 dargestellt. Im sechsten Schritt 260 wird basierend auf zumindest einer erkannten Defekt-Kante 81 ein Muster 62 ermittelt, das eine Mehrzahl an vorliegenden Korrelationswerten 83 umfasst. Dazu werden die Korrelationswerte 83 einer Mehrzahl an Kanten 81 gemeinsam, also im Wesentlichen gleichzeitig, mit dem jeweils zugehörigen Korrelationsschwellenwert 86 verglichen, die im bestimmungsgemäßen Betriebszustand Nullkorrelationen 85 sind. Aus den Beträgen der Korrelationswerte 83, und wie stark diese ggf. den zugehörigen Korrelationsschwellenwert 86 übersteigen, ergeben sich für den jeweiligen Defektfall charakteristische Muster 62. Die Muster 62 werden mittels einer Datenbank 60, mit der ein Musterabgleich 65 durchgeführt wird, im sechsten Schritt 260 erkannt. Dementsprechend wird durch dem Musterabgleich 65 eine Defektursache diagnostiziert. Wird ein Muster 62 im sechsten Schritt 260 erkannt, wird eine entsprechende Warnung 59 ausgegeben. Die Warnung 59 kann das Vorliegen des bestimmungswidrigen Betriebszustands der Anlage 10 anzeigen, eine Angabe über ein zur Defekt-Kante 81 zugehöriges defektes Gerät 12, und/oder eine Angabe über die erkannte Defektursache. Die Warnung 59 kann an eine Anzeigeeinheit 64 geleitet werden, die dazu ausgebildet ist, die Warnung 59 beispielsweise als visuelle oder akustische Warnung an einen Benutzer auszugeben. Alternativ oder ergänzend kann die Warnung 59 als elektronisches Warnsignal ausgebildet sein, das an eine Kommunikationsschnittstelle 66 ausgegeben wird, die mit einer nicht näher gezeigten übergeordneten Steuereinheit verbunden sein kann. Das Überwachungsverfahren 200 in FIG 2 wird durch ein Computerprogrammprodukt 70 umgesetzt, das auf einer Überwachungseinheit 75 ausgeführt wird. Das Computerprogrammprodukt 70 bildet eine Künstliche Intelligenz 99, die dazu eingesetzt wird, die als Gaschromatograph 11 ausgebildete Anlage 10 zu überwachen.

## Patentansprüche

1. Überwachungsverfahren (100) für eine Anlage (10) mit einer Mehrzahl an Geräten (12), die dazu ausgebildet sind, jeweils einen zugehörigen Messwert (15) und/oder einen Steuerbefehl (17) bereitzustellen, umfassend die Schritte:
a) Bereitstellen eines Eingangssignal-Arrays (30), das eine Mehrzahl an Zellen (32) für die Messwerte (15) und/oder Steuerbefehle (17) umfasst und Bereitstellen eines Überwachungsdaten-Arrays (50), das Zellen (52) umfasst, die mit den Zellen (32) des Eingangssignal-Arrays (30) korrespondieren;
b) Bereitstellen der Anlage (10) in einem aktiven Betriebszustand und Erfassen von Messwerten (15) und/oder Steuerbefehlen (17), die an das Eingangssignal-Array (30) übergeben werden;
c) Ermitteln eines System-Abweichungsparameters (55) anhand einer Mehrzahl an Inhalten von Zellen (52) des Überwachungsdaten-Arrays (50) und Erkennen eines bestimmungswidrigen Betriebszustands, wenn der System-Abweichungsparameter (55) einen einstellbaren System-Schwellenwert (57) betragsmäßig übersteigt;
d) Separates Ermitteln einer Mehrzahl an Zellen-Abweichungsparametern (47) anhand einer Mehrzahl an Inhalten von Zellen (32) im Eingangssignal-Array (30) und Inhalten von korrespondierenden Zellen (52) des Überwachungsdaten-Arrays (50) und Ermitteln einer Zelle (32, 52) als Defekt-Zelle (58), wenn ein zugehöriger Zellen-Abweichungsparameter (45) einen einstellbaren Zellen-Schwellenwert (47) betragsmäßig überteigt;
wobei eine Warnung (59) ausgegeben wird, wenn eine Defekt-Zelle (58) erkannt wird.

2. Überwachungsverfahren (100) nach Anspruch 1, **dadurch gekennzeichnet, dass** das Überwachungsverfahren (100) einen Schritt e) umfasst, in dem die ermittelte Defekt-Zelle (58) oder ein ermittelter Satz an Defekt-Zellen (58) erfasst wird und anhand eines Musterabgleichs (65) eine Defektursache erkannt wird.

3. Überwachungsverfahren (100) nach Anspruch 1 oder 2, **dadurch gekennzeichnet, dass** zumindest einer der Schritte a), b), c) und/oder d) mittels eines Autoencoders (42) durchgeführt wird und der System-Abweichungsparameter (55) ein Total-Reconstruction-Error ist.

4. Überwachungsverfahren (100) nach Anspruch 1 bis 3, **dadurch gekennzeichnet, dass** zumindest ein einstellbarer Zellen-Schwellenwert (47) als fester Schwellenwert oder als gleitender Schwellenwert ausgebildet ist.

5. Überwachungsverfahren (100) nach einem der Ansprüche 1 bis 4, **dadurch gekennzeichnet, dass** der Schritt b) ein Interpolieren umfasst, in dem für eine Mehrzahl an Messwerten (15) und/oder Steuerbefehlen (17) synchrone Messpunkte ermittelt werden.

6. Überwachungsverfahren (100) nach einem der Ansprüche 1 bis 5, **dadurch gekennzeichnet, dass** Eingangssignal-Array (30) und/oder das Überwachungsdaten-Array (50) Zellen (32, 52) umfasst, in denen Messwerte (15) und/oder Steuerbefehle (17) gespeichert werden, die einem einstellbaren Relevanz-Kriterium genügen.

7. Überwachungsverfahren (200) für eine Anlage (10) mit einer Mehrzahl an Geräten (12), die dazu geeignet sind, Knoten (82) in einem neuronalen Netz (80) zu bilden, das ein Zusammenwirken der Geräte (12) in der Anlage (10) abbildet, umfassend die Schritte:
a) Bereitstellen eines trainierten neuronalen Netzes (80), das eine Mehrzahl an Kanten (81) aufweist, die jeweils einer Null-Korrelation (85) oder einer Betriebskorrelation entsprechen und bei denen beim Trainieren ein Korrelationswert (83) ermittelt wird, der unterhalb eines einstellbaren Korrelationsschwellenwerts (86) liegt;
b) Bereitstellen der Anlage (10) in einem aktiven Betriebszustand und Erfassen von Messwerten (15) und/oder Steuerbefehlen (17), die jeweils mit zumindest einem Knoten (82) und/oder einer Kante (81) des neuronalen Netzes (80) korrespondieren;
c) Ermitteln eines Netz-Abweichungsparameters (94), der Korrelationswerte (83) einer Mehrzahl an Kanten (81) des neuronalen Netzes (80) kombiniert; wobei die Korrelationswerte (83) anhand der in Schritt b) erfassten Messwerte (15) und/oder Steuerbefehle (17) ermittelt werden;
d) Erkennen eines bestimmungswidrigen Betriebszustands der Anlage (10), wenn der Netz-Abweichungsparameter (94) einen einstellbaren Netz-Schwellenwert (95) betragsmäßig übersteigt;
wobei eine Warnung (59) ausgegeben wird, wenn der bestimmungswidrige Betriebszustand erkannt wird.

8. Überwachungsverfahren (200) nach Anspruch 7, **dadurch gekennzeichnet, dass** die Schritte b), c) und/oder d) für Betriebskorrelationen mit einer ersten Frequenz und für Nullkorrelationen (85) mit einer zweiten Frequenz durchgeführt wird.

9. Überwachungsverfahren (200) nach Anspruch 7 oder 8, **dadurch gekennzeichnet, dass** die Schritte b), c) und/oder d) in einem ersten Durchgang für Betriebskorrelationen durchgeführt werden und in einem zweiten Durchgang für NullKorrelationen (85), wenn im ersten Durchgang ein bestimmungswidriger Zustand erkannt wird.

10. Überwachungsverfahren (200) nach einem der Ansprüche 7 bis 9, **dadurch gekennzeichnet, dass** das Überwachungsverfahren (200) einen weiteren Schritt e) umfasst, in dem eine Kante (81) des neuronalen Netzes (80), die in einem bestimmungsgemäßen Betriebszustand einer Nullkorrelation (85) entspricht, als Defekt-Kante (87) erkannt wird, wenn deren Korrelationswert (83) den zugehörigen Korrelationsschwellenwert (86) betragsmäßig übersteigt.

11. Überwachungsverfahren (200) nach einem der Ansprüche 7 bis 10, **dadurch gekennzeichnet, dass** der Korrelationsschwellenwert (86) als fester Schwellenwert oder als gleitender Schwellenwert ausgebildet ist.

12. Überwachungsverfahren (100) nach einem der Ansprüche 1 bis 11, **dadurch gekennzeichnet, dass** das Überwachungsverfahren (200) einen weiteren Schritt f) umfasst, in basierend auf zumindest einer erkannten Defekt-Kante (87) ein Musterabgleich (65) durchgeführt wird und eine Defektursache erkannt wird.

13. Computerprogrammprodukt (70) zum Überwachen einer Anlage (10), die eine Mehrzahl an Geräten (12) aufweist, die zu einem Betrieb einer Anlage (10) zusammenwirken und zu denen Messwerte (15) und/oder Steuerbefehle (17) erfassbar sind und in einem Überwachungsverfahren (100, 200) verarbeitbar sind, **dadurch gekennzeichnet, dass** das Überwachungsverfahren (100, 200) nach einem der Ansprüche 1 bis 12 ausgebildet ist.

14. Überwachungseinheit (75) für eine Anlage (10), die eine Mehrzahl an Geräten (12) umfasst, die zu einem Betrieb der Anlage (10) zusammenwirken und zu denen Messwerte (15) und/oder Steuerbefehle (17) erfassbar sind, wobei die Überwachungseinheit (75) zum Empfangen und Verarbeiten der Messwerte (15) und/oder Steuerbefehle (17) ausgebildet ist und dazu ausgebildet ist, eine Warnung (59) auszugeben, **dadurch gekennzeichnet, dass** die Überwachungseinheit (75) zum Verarbeiten der Messwerte (15) und/oder Steuerbefehle (17) mit einem Computerprogrammprodukt (70) nach Anspruch 13 ausgestattet ist.

15. Gasanalysevorrichtung (11), umfassend eine Mehrzahl an Geräten (12) zum Aufbereiten und Vermessen einer Stoffprobe (22), die mit einer Überwachungseinheit (75) zum Erkennen eines bestimmungswidrigen Betriebszustands der Gasanalysevorrichtung (11) ausgebildet ist, **dadurch gekennzeichnet, dass** die Überwachungseinheit (75) gemäß Anspruch 14 ausgebildet ist.

16. Verwendung einer Künstlichen Intelligenz (99) zum Überwachen einer Gasanalysevorrichtung (11), wobei die Verwendung zumindest das Ausgeben einer Warnung (59) umfasst und die Künstliche Intelligenz (99) ein Überwachungsverfahren (100, 200) durchführt, **dadurch gekennzeichnet, dass** das Überwachungsverfahren (100, 200) nach einem der Ansprüche 1 bis 12 ausgebildet ist.
